# EUROPEAN PATENT SPECIFICATION

(11) **EP 4 117 558 B1**
(45) Date of publication and mention of the grant of the patent: **29.04.2026**
(21) Application number: 21709778.1
(22) Date of filing: 25.02.2021
(51) Int. Cl.: A61B 17/00, A61B 90/00, A61B 18/14, B25B 27/28

(54) **INSULATION OF AN ELECTROSURGICAL INSTRUMENT**
ISOLIERUNG EINES ELEKTROCHIRURGISCHEN INSTRUMENTS
ISOLATION D'UN INSTRUMENT ÉLECTROCHIRURGICAL

(30) Priority: 13.03.2020 GB 202003696
(43) Date of publication of application: 18.01.2023
(73) Proprietor: CMR Surgical Limited, Cambridge CB24 9NG (GB)
(72) Inventor: SHEARD, Joshua Philip, Cambridge Cambridgeshire CB24 9NG (GB); HEARN, Gareth Stephen, Cambridge Cambridgeshire CB24 9NG (GB); PIOTROWSKI, Michal, Cambridge Cambridgeshire CB24 9NG (GB)
(74) Representative: Slingsby Partners LLP
(86) International application number: PCT/GB2021/050489
(87) International publication number: WO 2021/181065

(56) References cited:
- US-A- 5 261 918
- US-A- 6 049 960
- US-A1- 2007 149 850
- US-A1- 2012 079 701

## Description

### Field of the invention

This invention relates to the insulation of an electrosurgical instrument, and in particular to a device for applying an insulating sleeve to an electrosurgical instrument.

### Background of the invention

Electrosurgery is a type of surgery that has been developed to replace traditional surgical methods and uses a high frequency electrical current to generate heat that provides a means to cut, dissect, fulgurate, ablate or shrink organic tissue. This method provides advantages such as increased cutting precision and minimised blood loss. Electrosurgery may be performed using one of two modalities; either a monopolar or bipolar modality. In monopolar electrosurgery the tissue is targeted using a singular probe electrode, whereas bipolar electrosurgery uses a bipolar device such as a pair of forceps.

Monopolar electrosurgery requires a firing drive cable that conducts an electrical current from an electrical supply to an end effector. In known monopolar electrosurgical systems, the end effector is supplied with current by a firing drive cable that conducts current from an electrical source to the end effector. In order for the end effector to be energised they must be electrically insulated from the other components located at the distal end of the electrosurgical equipment. One solution for insulating the end effector from the other electrical components is to encase them in an over-mould constructed from an electrically insulating material such as PEEK. A disadvantage associated with this over-mould is that it restricts the range of motion of the end effector. This limits the ability of the end effector to perform cold-cutting, in which it is actuated in the absence of any heat applied by the electrical current. In addition to this, design of the over-mould results in a high manufacturing complexity due to the alignment of the metallic parts of the instrument shaft over the plastic moulded parts, which results in an increase in part-to-part variability.

There is a need for a new mechanism for electrically insulating an electrosurgical instrument that overcomes the abovementioned disadvantages.

US 2012/079701 discloses a method for mounting a sheath onto the distal end of a surgical shaft instrument, in particular the tool tip of a dilation instrument. The method allows the sheath to be fastened on the instrument by the steps: a) fastening the sheath on an apparatus for mounting a sheath onto the distal end of a surgical shaft instrument; b) radially widening at least the proximal end of the sheath and inserting the distal end of the surgical shaft instrument into the widened sheath c) transferring the sheath from the apparatus to the distal end of the surgical shaft instrument; and d) removing the distal end of the surgical shaft instrument from the apparatus.

### Summary of the invention

The invention is defined in appended independent claims 1 and . Further embodiments are defined in appended dependent claims.

According to a first aspect, there is provided a device for applying an insulating sleeve to an electrosurgical instrument, the device comprising a housing, the housing comprising: a first end; a second end; a hollow channel extending from the first end to the second end, the channel being configured to receive an electrosurgical instrument comprising an end effector connected to a shaft by a distal articulation; a support structure internal to the housing; the support structure being configured to: support an insulating sleeve such that the insulating sleeve surrounds the electrosurgical instrument when the electrosurgical instrument is received by the channel; and when the electrosurgical instrument is advanced through the channel from the first end to the second end, release the insulating sleeve onto the electrosurgical instrument.

The channel may comprise a first portion and a second portion, wherein the first portion is narrower than the second portion.

The proximal end of the support structure may be located on a ledge that separates the first portion of the channel from the second portion of the channel.

The first portion of the channel may be circular in cross section, and the first portion may further comprise an opening located on the ledge of the support structure.

The support structure may comprise a plurality of protrusions surrounding the opening, the plurality of protrusions extending distally along an axis that is parallel to an axis along which the channel extends.

The plurality of protrusions may comprise a first set of protrusions and a second set of protrusions, wherein: each protrusion of the first set of protrusions extends to a first length; and each protrusion of the second set of protrusions extends to a second length that is shorter than the first length.

The length of each protrusion of the second set of protrusions may be between 45 to 50% of the length of each protrusion of the first set of protrusion.

The protrusions of the support structure may be uniformly positioned around the opening.

The first set of protrusions may comprise two diametrically opposing protrusions.

The second set of protrusions may comprise two diametrically opposing protrusions.

The housing may be formed of a first portion and a second portion, the first portion and the second portion being pivotally coupled.

The internal mechanisms of the first portion and the second portion may be identical.

The device may further comprise a hinge coupled to both the first portion and the second portion, the hinge enabling pivotal rotation of the first portion and the second portion of the housing relative to each other.

The device may further comprise a removeable applicator, and the housing may be further configured to receive the applicator.

The applicator may comprise: a first end that can be coupled to the second end of the device; a second end that can be positioned at the first end of the device and configured to receive an end effector; and a shaft extending between the first end and the second end.

The first end of the applicator may comprise a laterally extending member for releasably engaging the first end of the housing.

The end effector may be a pair of forceps and the second end of the applicator may comprise an aperture for housing the tips of the forceps when they are closed.

The diameter of the shaft of the applicator may be smaller than the diameter of the shaft of the electrosurgical instrument.

The shaft of the applicator may comprise a limiting ring, and the diameter of the limiting ring may be larger than the inner diameter of the insulating sleeve.

The protrusions may be angled such that the distance between two diametrically opposing protrusions is smaller than the shaft of the electrosurgical instrument.

According to a second aspect, there is provided a method for applying an insulating sleeve to an electrosurgical instrument, the electrosurgical instrument comprising an end effector connected to a shaft by a distal articulation at a distal end of the instrument, the method comprising: applying the insulating sleeve to a support structure of a device, the device comprising a housing with a first end, a second end, and a hollow channel extending from the first end to the second end, the support structure being internal to the housing and configured to interface with the insulating sleeve; progressing the shaft of the electrosurgical instrument through the hollow channel of the device; and engaging the shaft of the electrosurgical instrument with the support structure, which pushes the insulating sleeve off of the support structure and onto the electrosurgical instrument.

According to a third aspect, there is provided a method for removing an insulating sleeve from an electrosurgical instrument, the electrosurgical instrument comprising an end effector connected to a shaft by a distal articulation at the distal end of the instrument, the insulating sleeve being applied to the distal end of the electrosurgical instrument, the method comprising: positioning the electrosurgical instrument within a device, the device comprising a housing with a first end, a second end, a hollow channel extending from the first end to the second end, the channel being configured to receive the electrosurgical instrument, and a support structure internal to the housing, the support structure being further configured to interface with an insulating sleeve; progressing the first end of the housing of the device towards the distal end of the surgical instrument, such that the insulating sleeve is brought into contact with the support structure; and applying force to the housing such that, when the insulating sleeve is brought into contact with the support structure, the housing progresses with the support structure of the housing towards the distal end of the surgical instrument, until the insulating sleeve is removed from the distal end of the electrosurgical instrument.

### Detailed description

The present invention will now be described by way of example with reference to the accompanying drawings. In the drawings:
figure 1 illustrates an electrosurgical instrument to which electrical insulation is to be applied;
figure 2 illustrates an insulating sleeve for electrically insulating an electrosurgical instrument;
figure 3 illustrates an arrangement of the insulating sleeve of figure 2 when it is applied to the electrosurgical instrument of figure 1;
figure 4 illustrates a first view of a device for applying an insulating sleeve to an electrosurgical instrument;
figure 5 illustrates a view of the housing of the device illustrated in figure 4;
figure 6 illustrates a removeable applicator of the device of figure 4;
figure 7 illustrates the arrangement of the device housing of figure 4, the applicator of figure 6 and the insulating sleeve prior of figure 2 to assembly of the insulating sleeve onto the electrosurgical instrument;
figure 8 illustrates the process of applying an insulating sleeve to the electrosurgical instrument using the device of figures 4-7;
figure 9 illustrates the process of removing an insulating sleeve from the electrosurgical instrument using the device of figure 4.

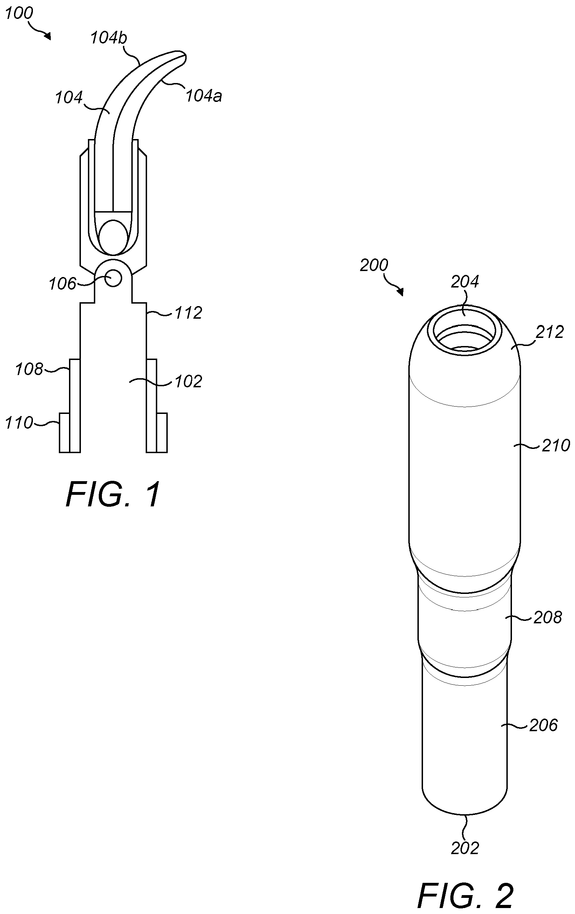

An example of the arrangement of a new electrosurgical instrument 100 is illustrated in figure 1. The instrument comprises a shaft 102 and an end effector 104. The shaft 102 is coupled at its distal end to the end effector 104, and at its proximal end to a surgical robot, which provides power to and controls the movement of the electrosurgical instrument 100. The surgical robot may comprise a console allowing the electrosurgical instrument 100 to be electrically manipulated by an operator. The surgical robot may further comprise a number of different electrosurgical instruments, both electrosurgical and non-electrosurgical.

The end effector 104 is configured for insertion into the body of the patient, and to perform a method of surgery. In the example illustrated in figure 1, the end effector 104 comprises a pair of curved blades 104a, 104b that form a pair of forceps. The forceps can be used to cut the organic tissues of a patient during a surgical procedure. It will be appreciated that, whilst the end effector in figure 1 is illustrated as a pair of forceps, an alternative end effector could be used in its place. The end effector 104 is coupled to the shaft 102 by an articulation 106. The articulation 106 allows the forceps to rotate about the shaft 102 in at least one degree of freedom.

During an electrosurgical procedure, power is supplied to the electrosurgical instrument 100 by the surgical robot. This power is supplied in the form of a high frequency, alternating electrical current. Current that is provided to the end effector 104 is passed through the shaft 102 of the electrosurgical instrument and the articulation 106. In known electrosurgical instruments, one or more drive cables located within the shaft are used to power the end effector. In contrast, for the arrangement illustrated in figure 1, the whole of the electrosurgical instrument 100, including the shaft 102, becomes live when current is provided to the end effector 104. The current heats the end effector 104 so that, when the end effector 104 is applied to organic tissue, the heated end effector 104 can coagulate and desiccate the tissue, which results in the occlusion of blood vessels and halting of bleeding. The end effector 104 may also be used to penetrate and dissect the tissue.

As, for the transfer of current through the electrosurgical instrument 100, the whole of the instrument 100 becomes live, it is necessary for the shaft 102 and the articulation 106 to be electrically insulated so as not to cause burns to the patient, either via direct contact or capacitively. To provide initial protection, the shaft 102 comprises two layers of insulation along its length. The first layer of insulation is a powder coating 108. The powder coating 108 may be an Ethylene Tetrafluoroethylene (ETFE) powder coating. The powder coating 108 extends to cover all but the distal end 112 of the shaft. The second layer of insulation comprises a heat shrink 110. The heat shrink may be a MT-1000 heat-shrink. The heat shrink 110 ends at the distal end of the shaft, but proximally to the end of the powder coating 108. The powder coating and heat shrink insulation cannot be applied to the articulation 106 of the electrosurgical instrument 100, as this would limit the range of motion of the articulation. Furthermore, the distal end of the shaft 112 and the articulation 106 must be capable of being effectively cleaned and sterilised between surgical procedures. At the same time, it is important that the distal end of the end effector 104 remains exposed. Where the end effector is a pair of forceps, it is important that the blades 104a, 104b of the forceps are exposed and are able to perform their full range of motion.

To provide electrical insulation to the distal end of the shaft 112 and the articulation 106 of the electrosurgical instrument 100, an insulating sleeve is provided. An example of the insulating sleeve 200 is illustrated in figure 2. The insulating sleeve comprises a first end 202, a second end 204, a plurality of portions 206, 208, 210, and a distal fillet 212. The first end 202 and the second end 204 each comprise an opening, and a hollow shaft extends through the insulating sleeve 200 between these two openings. Each portion of the insulating sleeve is for interfacing with a different part of the electrosurgical instrument 100 and may comprise a different internal and external diameter. Alternatively, each portion of the sleeve may have the same internal and external diameters and may be configured to stretch around the respective parts of the electrosurgical instrument that it is configured to cover. In the example illustrated in figure 2, the insulating sleeve comprises three portions. The insulating sleeve is also illustrated as being cylindrical in shape. It will be appreciated that alternative shapes of insulating sleeve may alternatively be provided.

The insulating sleeve 200 is formed of a material such as silicon, which is capable of resisting high temperatures that arise during electrosurgery. In addition to this, the material of the insulating sleeve is resistive to fatigue for the range of strains expected during end effector movement. The material of the insulating sleeve 200 is also elastic, so that it is able to expand to surround the electrosurgical instrument 100. The inner surface of the insulating sleeve 200 is coated to ensure a smooth finish. The coating of the inner surface is glutinous. This enables the surface of the electrosurgical instrument 100, which is polished, to stick to the inner surface of the insulating sleeve 200. Thus, high friction between the surface of the electrosurgical instrument 100 and the insulating sleeve 200 is ensured.

The first portion 206 of the insulating sleeve 200 is located at a proximal end of the sleeve. The first portion 206 is configured to interface with the powder coating 108 located on the outer surface of the shaft 102. The inner diameter of the first portion 206 of the insulating sleeve 200 is structured so that, when it is not expanded, it is smaller than the outer diameter of the portion of the shaft comprising the powder coating 108. For example, if the outer diameter of the instrument shaft comprising the powder coating is 6.8mm, the inner diameter of the first portion 206 of the insulating sleeve may be 4.2mm. As the insulating sleeve is made of an elastic material, the inner diameter of the first portion 206 of the sleeve can be stretched to expand its inner diameter such that it can be applied to the electrosurgical instrument.

The smaller inner diameter of the first portion 206 relative to the portion of the shaft comprising the powder coating 108 ensures a compression fit between the sleeve 200 and the instrument shaft 102. Thus, the insulating sleeve 200 can be well positioned on the instrument shaft 102 during assembly, such that displacement of the sleeve 200 relative to the shaft 102 during surgical use is prevented. This is advantageous as it minimises the risk of the sleeve 200 falling onto the surgical site during use. The inner diameter of the first portion of the sleeve 206 may be between 60% and 63% of the diameter of the instrument shaft 102. The inner diameter of the first portion of the sleeve 206 may be between 80 and 88% of its outer diameter. In the example where the inner diameter of the first portion 206 is 4.2mm, the outer diameter of the first portion may be 5mm. In this example, the inner diameter of the second portion 206 will be 84% of the diameter of the outer diameter of the second portion. The inner and outer diameters of the first portion 206 are selected such that the thickness of its walls allows for the first portion 206 to stretch and cover the shaft 102 without tearing.

The second portion 208 of the insulating sleeve 200 is connected to and located distally of the first portion 206 of the sleeve. The second portion 208 is configured to interface with the outer surface of the distal end of the shaft 112 that does not comprise any alternative form of insulation. The inner diameter of the second portion 208 is larger than the inner diameter of the first portion 206. The inner diameter of the second portion 208 may also be larger than the outer diameter of the shaft 102. For example, if the outer diameter of the shaft is 6.8mm, the inner diameter of the second portion may be 4.5mm. As it is wider in diameter than the first portion 206, the second portion 208 (together with the third portion 210) is easier to position over the shaft after the first portion 206 has been positioned. The inner diameter of the second portion of the sleeve 208 may be between 65% and a 68% of the diameter of the instrument shaft 102. The inner diameter of the second portion of the sleeve 208 may be between 78 and 84% of the outer diameter of the second portion 208 of the sleeve. In the example where the inner diameter of the second portion 206 is 4.5mm, the outer diameter of the first portion may be 5.56mm. In this example, the inner diameter of the second portion 206 will be 81% of the diameter of the outer diameter of the second portion.

The third portion 210 of the insulating sleeve 200 is connected to and located distally of the second portion 208 of the sleeve. The third portion 210 is configured to interface with the articulation 106 of the electrosurgical instrument 100. The third portion 210 therefore has a larger inner diameter than both the first portion 206 and the second portion 208, so that it is able to encapsulate the articulation 106. For example, where the inner diameters of the first and second portions are 4.2mm and 4.5mm respectively, the inner diameter of the third portion may be 5.9mm. The inner diameter of the third portion of the sleeve 210 may be between 85% and 88% of the diameter of the instrument shaft 102. The inner diameter of the third portion of the sleeve 210 may be between 84 and 89% of the outer diameter of the third portion 210 of the sleeve. Where the inner diameter of the third portion is 5.9mm, its outer diameter may be 6.8mm. In this example, the inner diameter of the second portion 206 will be 87% of the diameter of the outer diameter of the second portion.

The distal end of the third portion 210 is further connected to a fillet 212. The fillet 212 is located distally of the third portion 210, where the second portion 208 is located proximally of the third portion 210. The fillet decreases in radius as it extends away from the third portion 210 such that the inner diameter the distal end of the sleeve, which opposes the proximal end, is narrower than the inner diameter of the third portion 210. In contrast, the thickness of the fillet 212 increases as it extends distally. The fillet 212 is configured to interface with the end effector 104 of the electrosurgical instrument. Where the electrosurgical instrument is a pair of forceps, the fillet 212 is configured to couple, at its distal end, to the blades 104a, 104b, of the forceps. The increased thickness of the fillet 212 at its distal end results in a thicker ring that surrounds the electrosurgical instrument 100, which enables attachment of the insulating sleeve 200 to the instrument.

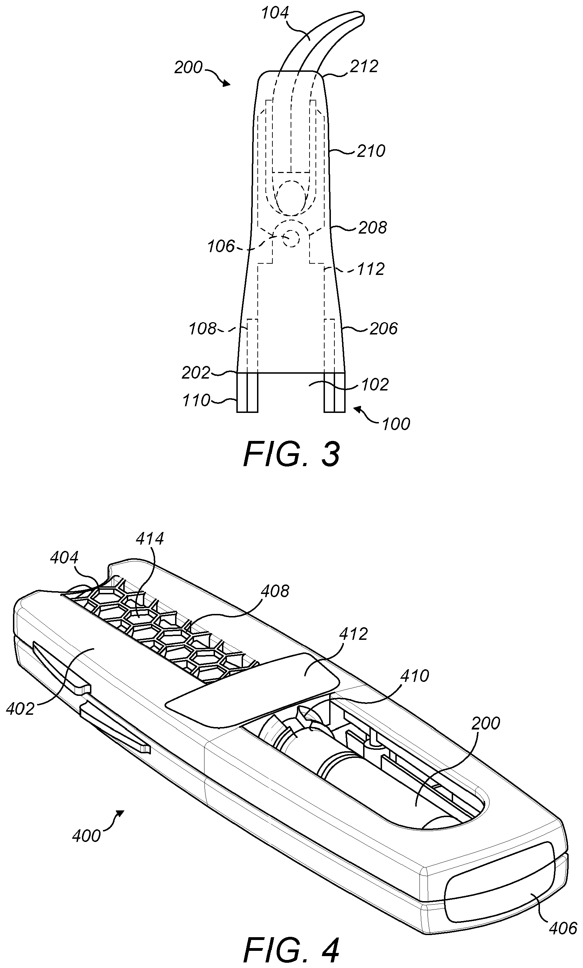

Figure 3 illustrates an exemplary arrangement of the insulating sleeve 200 placed over the electrosurgical instrument 100. In this arrangement the first end 202 of the sleeve interfaces with the distal end of the heat shrink 110 that insulates the shaft 102. The first portion 206 of the sleeve is positioned over and interfaces with the powder coating 108. The second portion 208 of the sleeve overlies the distal part 112 of the shaft. The third portion 210 of the sleeve overlies the articulation 106 and the fillet 212 overlies a portion of the end effector 104. Where the end effector is a pair of forceps, the fillet 212 overlies a portion of the blades of the end effector 104.

The first portion 206 of the sleeve does not necessarily have to directly abut the distal end of the heat shrink 110. However, to ensure that an operator of the electrosurgical instrument is protected from electrical shocks induced by the live system, it is important that the creepage and clearance distances between the proximal end of the insulating sleeve 200 and the live portions of the shaft 102 are maintained. The creepage distance is the shortest distance along the surface of a solid insulating material between two conductive parts. Clearance distance is the shortest distance in air between two conductive parts.

To ensure that both the creepage and clearance distances are maintained, an appropriate positioning of the insulating sleeve 200 over the electrosurgical instrument 100 is essential. This is because it may be difficult to reposition the insulating sleeve 200 once it has been placed on the instrument 100. The insulating sleeve 200 can be positioned on the shaft 102 of the instrument manually. However, this process incurs considerable time and effort. Manual positioning of the sleeve 200 may also result in perforation of the sleeve with the tip of the end effector 104 or harm caused by the tip of the end effector if the sleeve is displaced.

A device has been provided to ensure the correct application of an insulating sleeve onto the shaft. An example of this device 400 is illustrated in figure 4. The device 400 comprises a housing 402 that is configured to house an internal mechanism. The housing 402 is small and ergonomically arranged so that it can fit within the hand of a user. In the example illustrated in figure 4, the housing has a generally ovular shape. The housing 402 of the device 400 may be formed of a thermoelastic polymer, such as polypropylene.

The housing comprises a first end 404, a second end 406, and a hollow channel 408 that extends between the first end 404 and the second end 406. The dimensions of the channel 408 are such that it is configured to receive an electrosurgical instrument as illustrated in figure 1, for example. As illustrated in figure 1, the electrosurgical instrument 100 comprises a shaft 102, a distal articulation 106 and an end effector 104 that is located distally of the distal articulation. The smallest diameter of the channel 408 must therefore be larger than the largest diameter of the electrosurgical instrument 100.

The housing 402 further comprises a support structure 410 located internally of the housing. The support structure is configured to support an insulating sleeve as illustrated in figure 2, for example. The support structure 410 is configured to mount the insulating sleeve 200 such that the sleeve surrounds the electrosurgical instrument 100 when the instrument is positioned within the channel 408. The arrangement of the housing 402 is such that, when an electrosurgical instrument 100 is advanced through the channel from the first end 404 to the second end 406, the insulating sleeve is released onto the instrument by the support structure 410. In figure 4, the insulating sleeve 200 is illustrated as mounted on the support structure 410.

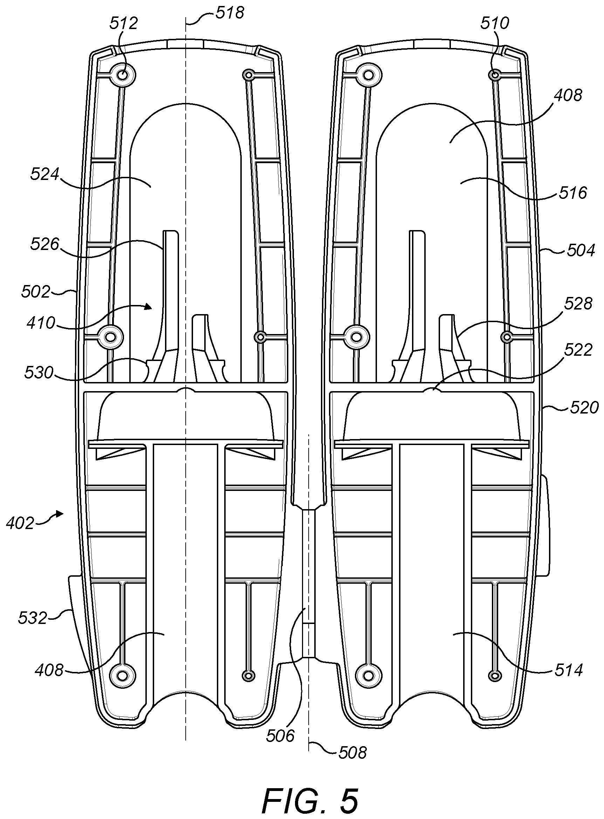

Figure 5 illustrates an alternative view of the housing 402 of the device 400. The housing 402 comprises a first part 502 and a second part 504. Preferably, the internal mechanisms of the two parts 502, 504 are identical. The two parts 502, 504 are pivotally coupled together. In figure 5, the two parts 502, 504 are coupled together by a hinge 506. The hinge 506 has a first axis 508 about which the first part 502 and the second part 504 are able to rotate. By forming the housing 402 from two parts 502, 504, the housing 402 can be opened to enable the electrosurgical instrument 100 to be removed from the housing once the insulating sleeve 200 has been applied to the instrument.

The first and second parts 502, 504 can be connected together using a latching mechanism. A first portion 510 of the latching mechanism may be located on the first part 502 of the housing 402, and a second portion 512 of the latching mechanism may be located on the second part 504 of the housing 402. The latching mechanism may be secured when the two parts 502, 504 of the housing are rotated about the first axis 508 of the hinge 506 so that they interconnect. The latching mechanism may be released to separate the first part 502 and the second part 504 by applying force to either the first or second portion of the latching mechanism. This application of force may comprise sliding, pushing or pulling either the first portion 510 or the second portion 512 of the latching mechanism to separate these components from each other.

In one example, the first portion 510 of the latching mechanism comprises a plurality of pins located in a first position within one of the portions the housing 402 of the device. In the same example, the second portion 512 of the latching mechanism comprises a plurality of holes located in a corresponding position to the first position of the pins located in the first portion 402. Latching of the first part 502 of the housing to the second part 504 of the housing is therefore achieved by insertion of the pins into their corresponding holes. In another example, as illustrated in figure 5, both the first part 502 and the second part 504 of the housing comprise a plurality of pins and a plurality of holes corresponding to those pins.

The channel 408 of the housing 402 is formed from recesses located in both the first and second parts 502, 504 of the housing 402. When these parts of the housing are interconnected, the channel 408 forms a closed passage located within the housing 402. The channel 408 further comprises a first portion 514 and a second portion 516. The first portion 514 of the channel extends along the length of the housing 402 from its first end 404 along a central axis 518. In figure 5, the axis 518 is illustrated as intersecting the channel 408 in the first part 502 of the housing. However, it will be appreciated that, when the housing 402 is closed, the axis 518 is located in the middle of the first part 502 and the second part 504 of the housing. The second portion 516 of the channel extends in a direction opposing the direction of extension of the first portion 514 of the channel along the length of the housing 402 from its second end 406. The first portion 514 and the second portion 516 of the channel intersect at a ledge 520. A first part of the ledge 520 is located in the first part 502 of the housing, and a second part of the ledge 520 is located in the second part 504 of the housing.

The ledge 520 comprises an opening 522 interconnecting the first portion 514 and the second portion 516 of the channel 408. The channel 408 may be circular in cross section, such that both the first portion 514 and the second portion 516 of the channel form a cylinder. The first and second portions 514, 516 may alternatively have a cross section formed from any other geometric shape. The first and second portions 514, 516 of the channel 408 may have the same diameter. Alternatively, the second portion 516 of the channel 408 may have a diameter that is larger than the diameter of the first portion 514 of the channel.

The support structure 410 of the housing 402 is located on the ledge 520 that connects the first portion 514 and the second portion 516 of the channel. More precisely, the proximal end of the support structure 410 is located on and connected to the ledge 520. The support structure 410 may be formed of a plurality of protrusions 524. The plurality of protrusions 524 may surround the opening 522 located on the ledge 520. The proximal ends of the plurality of protrusions 524 may thus surround the first portion 514 of the channel.

In the example illustrated in figure 5, each protrusion in the plurality of protrusions 524 extends along an axis that is parallel to the axis 518 of the channel. The plurality of protrusions 524 extends towards the second end 406 of the housing 402. The plurality of protrusions 524 further comprises a first set of protrusions 526 and a second set of protrusions 528. Each protrusion of the first set of protrusions extends to a first length from the ledge 520 towards the second end 406 of the housing 402. Each protrusion of the second set of protrusions 528 extends to a second length that is shorter than the first length.

The length of each protrusion of the second set of protrusions 528 may be between 45 and 50% of the length of each protrusion of the first set of protrusions 526. In one example, the length of each protrusion of the second set of protrusions 528 is 47% of the length of each protrusion of the first set of protrusions 526. The length of each protrusion of the first set of protrusions 526 may be between 20 and 25% of the length of the housing 402 of the device. In one example, the length of each protrusion of the first set of protrusions 526 is 21% of the length of the housing 402 of the device. The length of each protrusion of the second set of protrusions 528 may be between 8 and 13% of the length of the housing 402 of the device. In one example, the length of each protrusion of the second set of protrusions 528 is 10% of the length of the housing 402 of the device. The lengths of the protrusions of the first and second sets of protrusions relative to each other, and to the overall length of the housing, are selected to optimise the process of applying the sleeve 200 to the surgical instrument 100.

Each protrusion of the plurality protrusions 524 of the support structure 410 may be uniformly positioned around the opening 522. That is, each protrusion of the first set of protrusions 526 may be uniformly spaced around the opening 522 in the ledge 520 relative to the other protrusions of that set of protrusions. Correspondingly, each protrusion of the second set of protrusions 528 may be uniformly spaced around the opening 522 in the ledge 520 relative to the other protrusions of that set of protrusions. As mentioned above, the housing 402 of the housing 402 is formed from a first part 502 and a second part 504. Preferably, the internal mechanisms of the first part 502 and the second part 504 are identical. Thus, the first and second parts 502, 504, may comprise an equal number of protrusions. The protrusions may therefore be equally spaced on the parts of the ledge 520 that are formed by both the first 502 and the second 504 part.

In the example illustrated in figure 5, both the first set 526 and the second set 528 of protrusions comprise two protrusions. A first protrusion of the first set of protrusions 526 is located on the part of the ledge 520 formed by the first part 502 of the housing, A second protrusion of the first set of protrusions 526 is located on the part of the ledge 520 formed by the second part 504 of the housing. Correspondingly, a first protrusion of the second set of protrusions 528 is located on the part of the ledge 520 formed by the first part 502 of the housing, and a second protrusion of the second set of protrusions 528 is located on the part of the ledge 520 formed by the second part 504 of the housing. In this example, the first and second protrusions of the first set of protrusions 526 are diametrically opposing protrusions. The first and second protrusions of the second set of protrusions 528 are diametrically opposing protrusions.

The protrusions of the plurality of protrusions 524 are structured so that, as they extend in a longitudinal direction away from the ledge 520, they are angled inwards. Hence, the diameter that is formed by the distance between the tips of each protrusion of the first set of protrusions 524 is smaller than the diameter of the opening 522. Likewise, the diameter that is formed by the distance between the tips of each protrusion of the second set of protrusions 528 is smaller than the diameter of the opening 522. The diameter that is formed by the tips of each protrusion of the first set of protrusions 526 may be different to (i.e. either smaller than or larger than) the diameter that is formed by the tips of each protrusion of the second set of protrusions 528. Alternatively, the diameters of the protrusions of the first set of protrusions 526 and the second set of protrusions 528 may be the same.

The protrusions of the plurality of protrusions 524 are constructed from a flexible plastic material. Each protrusion of the plurality of protrusions may further comprise a step 530. The step may be located at the proximal end of each protrusion. The proximal end of each protrusion is the end that is closest to the ledge 520. The step 530 of each protrusion provides an increase in diameter of the protrusion at the location of the step over the diameter of the remaining proximal end of the protrusion.

The function of the plurality of protrusions 526 is to locate an insulating sleeve 200 on the housing 402 so that is arranged to be placed over the electrosurgical instrument 100. When the first part 502 and the second part 504 of the housing 402 are arranged so that they intersect, and the housing 402 is closed, the protrusions are disposed in a circular arrangement around the opening 522 on the ledge 520 of the housing 402. Thus, each step 530 of a protrusion in the plurality of protrusions 524 provides a mounting surface on which the insulating sleeve 200 can be located. More specifically, the first portion 206 of the insulating sleeve is positioned on the steps of the plurality of protrusions. The position of the first portion 206 of the insulating sleeve 200 is therefore maintained by the plurality of protrusions 524.

The diameter of the circle formed by the distance between the steps 530 of the plurality of protrusions is larger than the inner diameter of the first portion 206 of the insulating sleeve 200. Thus, the plurality of protrusions 524 is able to stretch the first portion of the sleeve so that this inner diameter is increased. The inner diameter of the first portion 206 is stretched such that it is larger than the diameter of the shaft 102 of an electrosurgical instrument onto which the insulating sleeve 200 is to be applied.

The housing 402 may be arranged so that it is ergonomically suited for use by an operator. The housing 402 may comprise one or more extruded portions 412 that are suited to be held by the hand of a user. As mentioned above, the overall size of the housing 402 is such that it fits within the hand of a user. The housing 402 may further comprise a honeycomb structure 414 on its exterior. The honeycomb structure 414 provides enhanced gripping during use of the device 400. The housing 502 may further comprise one or more auxiliary elements 532 to facilitate the opening of the housing 402. In the exemplary arrangement illustrated in figure 5, these auxiliary elements are located close to the second end 406 of the housing 402. However, it may be appreciated that these elements could be located in any alternative location along the edge of the first and second parts 502, 504 of the housing 402.

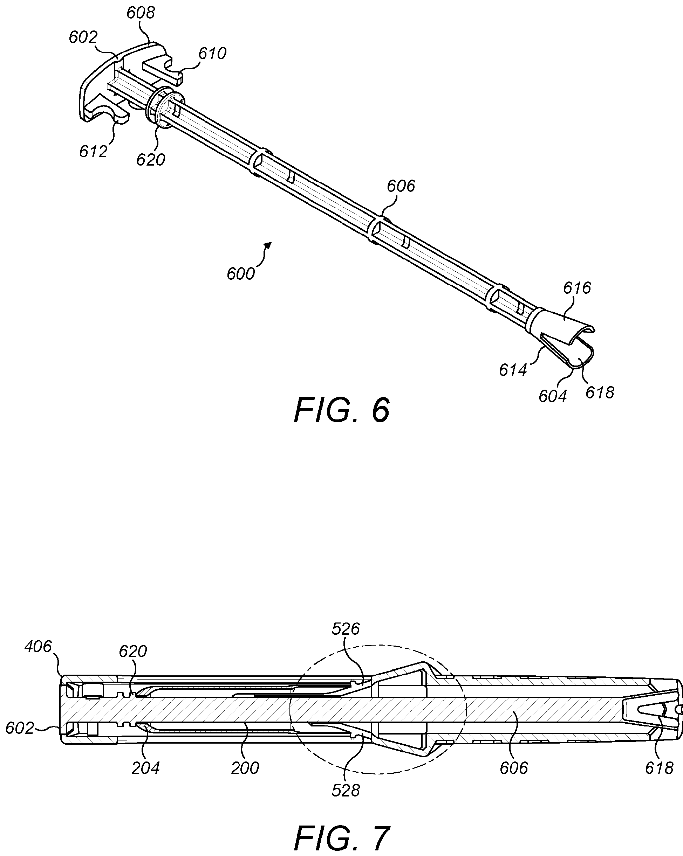

The device 400 further comprises a removeable applicator which is used to guide the electrosurgical instrument 100 through the housing 402 so that the sleeve can be provided to the instrument. The removeable applicator is illustrated in figure 6.

The applicator 600 illustrated in figure 6 comprises a first end 602, a second end 604 and a shaft 606 extending between the first end and the second end. The first end 602 is configured such that it can be coupled to the second end 406 of the housing 402. The second end 604 is configured to be positioned at the first end 404 of the housing 402 and to receive the distal end of the end effector 104 during its use.

The first end 602 of the applicator 600 comprises a laterally extending member 608. That is, the laterally extending member 608 extends in a direction that is substantially perpendicular to the longitudinal axis of the shaft 606 of the applicator. The laterally extending member may be symmetrical on either side of a line that is formed by the longitudinal axis of the shaft 606. The laterally extending member 608 is configured to releasably engage the second end 406 of the housing 402. The engagement between the second end of the housing 406 and the laterally extending member 608 is ensured when the first part 502 and the second part 504 of the housing 402 have intersected to close the channel 408 of the housing 402.

The applicator 600 further comprises two latching components 610, 612. The latching components 610, 612 are located at either end of the laterally extending member 608 and extend towards the second end 604 of the applicator. The latching components 610, 612 are configured to engage with an opening located at the second end 406 of the housing 402. The housing 402 may therefore comprise a recess that is complementary to the shape of the laterally extending member 608.

The second end 604 of the applicator 600 may further comprise two portions 614, 616. Each of the two portions 614, 616 is preferably formed from a rigid plastic, and comprises a proximal end that is connected to the shaft 606 and a distal end located that opposes the proximal end. The two portions 614, 616 may configured to separate from each other at their distal ends. The separation of these portions provides an aperture 618 at the distal part of the second end 604 of the applicator. The aperture 618 is configured to interface with the end effector 104 of an electrosurgical instrument 100. For example, where the end effector 104 is a pair of forceps, the aperture 618 is configured to interface with and house the distal ends of the blades 104a, 104b of the forceps when they are closed. When the end effector 104 interfaces with the aperture 618, the longitudinal axis of the shaft 102 of the electrosurgical instrument 100 is aligned with the longitudinal axis of the shaft 606 of the applicator 600. Thus, when the applicator 600 is placed within the housing 402 of the device and the end effector 104 is engaged with the aperture 618, the applicator 600 enables the electrosurgical instrument 100 to be guided though the housing 402 in alignment with the applicator. The electrosurgical instrument 100 can therefore be guided accurately through the housing 402.

The diameter of the shaft 606 of the applicator 600 is smaller than the diameter of the shaft 102 of the electrosurgical instrument 100 to be used in combination with that applicator. The diameter of the shaft 606 of the applicator is similar to the diameter that is formed by the distance between the tips of each protrusion of both the first set of protrusions 526 and the second set of protrusions 528. The shaft 606 is configured to pass through both the first portion 514 and the second portion 516 of the channel 408 of housing 402 without interfering with the opening 522 or the support structure 410 of housing 402. The diameter of the shaft 606 of the applicator is also smaller than the diameter that is formed by the distance between the tips of each protrusion of both the first set of protrusions 526 and the second set of protrusions 528. Thus, the shaft 606 of the applicator can be progressed through the housing 402 without altering the positioning of the protrusions.

The applicator 600 further comprises a limiting ring 620 located on its shaft 606. The diameter of the limiting ring 620 is larger than the diameter of the shaft 606. The diameter of the limiting ring 620 is also larger than the inner diameter of the fillet 212 of the insulating sleeve 200. To position the insulating sleeve 200 on the applicator 600, the first end 204 of the sleeve is slid onto the second end 604 of the applicator and is pushed up the shaft 606 until the filet 212 abuts the limiting ring 620. When the fillet 212 abuts the limiting ring 620, it cannot be slid any further up the applicator 600. Thus, the limiting ring 620 maintains the position of the insulating sleeve 200 on the shaft 606 before it is placed within the housing 402. That is, the limiting ring 620 prevents the sleeve from being displaced further towards the first end 602 of the applicator on the shaft 606 during storage due to temperature or time dependent deformations in the sleeve. This is important as it ultimately ensures that the insulating sleeve 200 is placed in the desired position on the electrosurgical instrument 100.

Figure 7 illustrates the arrangement of the housing 402, the removeable applicator 600 and the insulating sleeve 200 prior to application of the insulting sleeve 200 onto an electrosurgical instrument 100. To form this arrangement, the insulating sleeve 200 is first placed on the applicator 600 such that its fillet 212 abuts the limiting ring 620. The applicator 600 is then inserted into the housing 402. As the insulating sleeve 200 is arranged around the circumference of the shaft 606 of the applicator 600, the applicator can be used to align the insulating sleeve to the housing 402.

In the arrangement illustrated in figure 7, the first end 602 of the applicator is coupled to the second end 406 of housing 402. That is, the latching components 610, 612 are engaging with a corresponding opening in the housing 402. The second end 204 of the insulating sleeve 200 is engaged with the limiting ring 620 of the applicator 600. The first end 202 of the insulating sleeve 200 is abutting the steps 530 located on the first 526 and second 528 sets of the plurality of protrusions 524. The insulating sleeve 200 is therefore mounted on the housing 402. The aperture 618 located at the second end 604 of the applicator 600 is visible. The second end of the aperture 604 is located at the first end 404 of housing 402. The aperture 618 is configured to receive the distal end of the end effector 104.

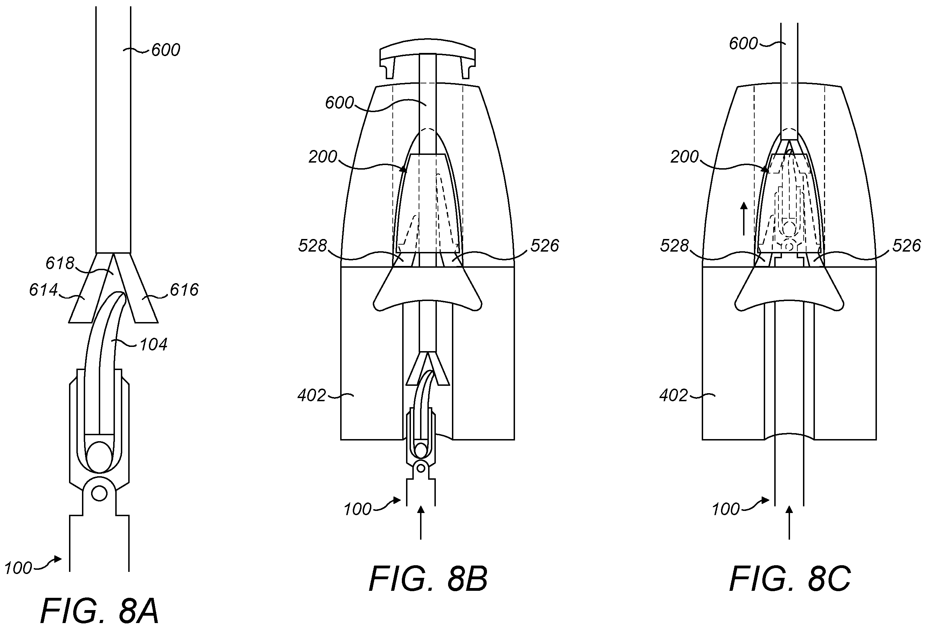

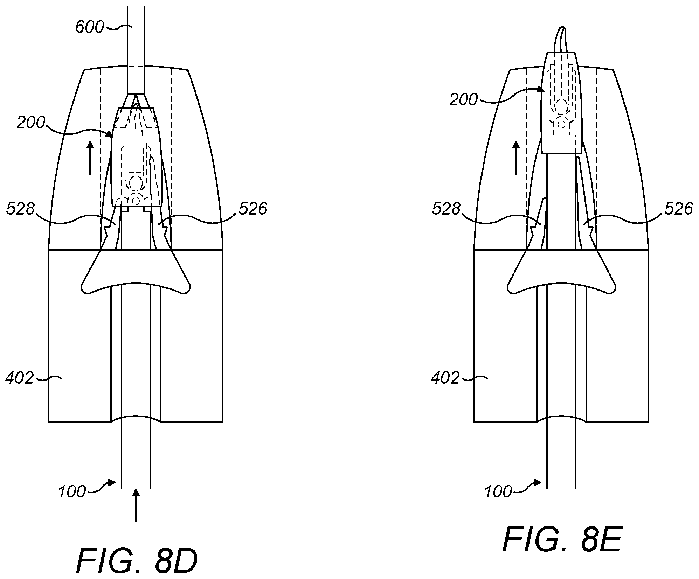

Figure 8 illustrates the method of applying an insulating sleeve 200 to an electrosurgical instrument 100 using the device illustrated in figures 4-7. In order to commence this method, the insulating sleeve 200 is first arranged so that it is mounted on the support structure 410 of the housing 402. More specifically, the insulating sleeve 200 is mounted on the steps 530 of the plurality of protrusions 524 of the support structure 410, such that the first end 202 of the insulating sleeve 200 interfaces with these steps. This arrangement is illustrated in the overview of the device that is provided in figure 4.

The insulating sleeve 200 may be mounted to the support structure 410 using the applicator 600. The insulating sleeve 200 may initially be mounted to the applicator 600 so that its second end 204 (i.e. the distal end of the fillet 212) interfaces with the surface of the limiting ring 620. The applicator 600 may then be placed within the channel 408 of the housing 402 by pivoting the first part 502 and the second part 504 of the housing about axis 508 of its hinge 506 so that they are separated. The applicator 600 can be placed into the recess that forms the channel 408 on either the first 502 or second 504 part of the housing, and the first and second parts can then be closed together. The first portion 206 of the insulating sleeve 200 is thus positioned on the support structure 410. The second portion 208 or any further portion of the insulating sleeve 200 may also be placed over the support structure 410. As mentioned above, the first end 202 of the insulating sleeve 200 should abut the steps 530 on the plurality of protrusions 524 of the support structure 410. When the insulating sleeve 200 is positioned in this way, the laterally extending member 608 of the applicator 600 interfaces with the second end 406 of the housing. The latching components 610, 612 are therefore engaged with the second end 406 of the housing 402.

The method of applying an insulating sleeve 200 to an electrosurgical instrument 100 begins at step 8A when the distal end of the electrosurgical instrument 100 is introduced into the second end 604 of the applicator 600. More specifically, the end effector 104 of the electrosurgical instrument 100 may be introduced into the aperture 618 of the applicator 600. Where the end effector 104 is a pair of forceps, the closed blades 104a, 104b of the forceps may be inserted into the aperture 618. When the end effector 104 is engaged with the aperture 618, the longitudinal axis of the shaft 102 of the surgical instrument 100 is aligned with the longitudinal axis of the applicator 600.

As mentioned above, the first and second portions 614, 616 are preferably formed of a rigid plastic, and the aperture 618 is provided by the separation of these rigid portions at their distal ends. In an alternative example, the first and section portions 614, 616 may be formed of a flexible plastic. In this example, the distal ends of the first and second portions 614, 616 adjoin each other when no end effector has been applied to the applicator. The insertion of an end effector pushes the distal ends of these portions 614, 616 apart to reveal the aperture 618.

At step 8B, the applicator 600 is continuously progressed through the housing 402 by pushing the shaft 102 of the electrosurgical instrument 100 along the longitudinal axis 518 of the channel 408. The insulating sleeve 200 and the housing 402 remain stationary. The progression of the applicator 600 through the housing 402 may be done manually. As the end effector 104 of the electrosurgical instrument 100 is engaged with the aperture 618 of the applicator 600, pushing the electrosurgical instrument 100 forces the applicator 600 to progress through the housing 402. The diameter of the shaft 606 of the applicator 600 is configured such that it can pass through the inner diameters of the insulating sleeve 200 when the insulating sleeve 200 is positioned on the support structure 410 of the housing 402.

At step 8C, the second end of the applicator 604 passes through the opening 520 and the support structure 410. Up until this point, the insulating sleeve 200 is still positioned on the support structure 410. The shaft 102 of the electrosurgical instrument is significantly wider than both the applicator 600 and the distance between the protrusions of the support structure 410 so, as the shaft 102 is progressed through the opening 520, it engages with and pushes the plurality of protrusions outwards. This, in turn, expands the inner diameter of the first portion 206 of the sleeve that is positioned on the support structure 410. This expansion in the inner diameter of the first portion results in an increase in friction that pushes the sleeve upwards towards the second end 406 of the housing 402. As mentioned above, the diameter that is formed by the distance between the tips of each protrusion of the first and second sets of protrusions is smaller than the diameter of the opening 520. Thus, as the electrosurgical instrument 100 and the applicator 600 continue to advance through the housing 402, the insulating sleeve 200 is pushed off of the steps 530 of the plurality of protrusions 524 of the support structure 410 and onto the shaft of the electrosurgical instrument 100 through the engagement of the support structure with the shaft 102. When the second end 604 reaches the fillet 212 of the insulating sleeve, the engagement between the second end and the fillet results in additional friction which assists with completing the adherence of the sleeve 200 to the instrument 100.

At step 8D, the insulating sleeve 200 has advanced such that its first end has passed the tips of the protrusions of the second set of protrusions 528. At this point, the insulating sleeve 200 is only interfacing with the support structure 410 by means of the protrusions of the first set of protrusions 526. The protrusions of the first set of protrusions 526 apply less force to the insulating sleeve than the combination of the first and second sets of protrusions 524. For this reason, less force is required at this stage to continue to progress the electrosurgical instrument 100 through the housing 402. The inner diameter of the portion of the insulating sleeve 200 that is still attached to the support structure 410 is also reduced.

At step 8E, the insulating sleeve 200 is further advanced such that it is removed from the tips of the protrusions of the first set of protrusions 526. The sleeve 200 is therefore fully applied to the electrosurgical instrument 100. The diameter of the sleeve 200 has reduced so that it adheres to the electrosurgical instrument 100. The applicator 600 can be removed from the insulating sleeve 200 after assembly by pulling on the first end 602 of the applicator so that the first and second portions 613, 616 of the applicator are forced out of the inner diameter of the third portion 210 of the sleeve.

Once the insulating sleeve 200 has been applied to the electrosurgical instrument 100, the housing 402 may then be removed from the instrument. This is done by releasing the latching mechanism of the housing and rotating the first and second parts 502, 504 of the housing away from each other about the axis 508 of the hinge 506. The electrosurgical instrument 100 may then be manually removed from the housing 402. If necessary, the insulating sleeve 200 can be manually adjusted on electrosurgical instrument 100 after its application, to ensure that its positioning is correct. The housing 402 may be retained for future use. The housing 402 can be applied to and removed from the electrosurgical instrument 100 whilst the instrument is coupled to a surgical robot.

As previously mentioned, the plurality of protrusions 524 in the support structure 410 comprises a first set of protrusions 526 and a second set of protrusions 528. The protrusions of the second set of protrusions 528 are shorter than the protrusions of the first set of protrusions 526. This is advantageous as it enables the insulating sleeve 200 to be supported by all of the protrusions of the support structure 410 before the electrosurgical instrument 100 is passed through the sleeve. This means that a suitable amount of force is applied to the inner diameter of the first portion 206 of the insulating sleeve, and that it can be held so that it is wide enough for the shaft 606 of the applicator 600 to pass through. Once the shaft 606 has passed through the insulating sleeve 200, it is easier to continue to push the electrosurgical instrument 100 through using a smaller number of protrusions. Thus, when the insulating sleeve 200 is advanced off of the support structure 410 its first end passes the tips of the protrusions of the second set of protrusions 528 and is supported only by the ends of the protrusions of the second set of protrusions 526. In this way, the amount of force that is applied to widen the inner diameter of the first portion 206 of the insulating sleeve 200 is varied to correspond to the path of the insulating sleeve 200 as it is applied to the electrosurgical instrument 100.

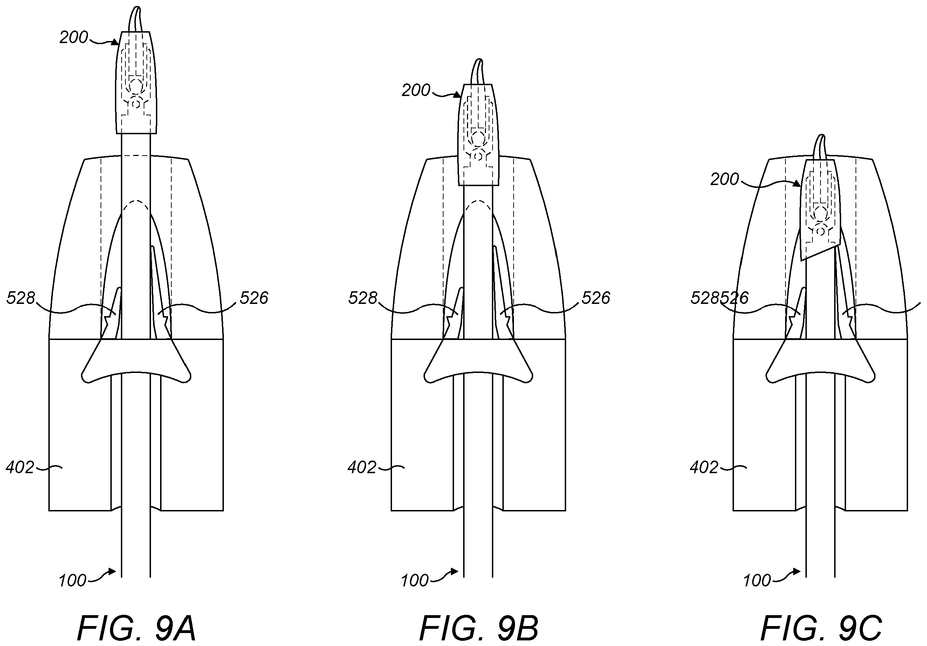

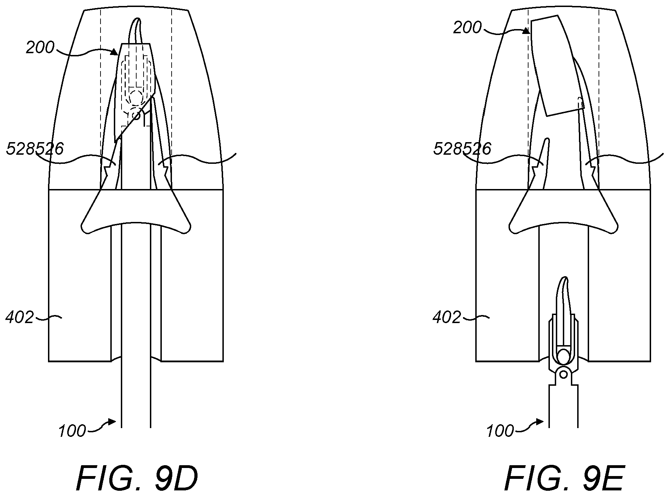

The housing 402 of the device 400 may also be used to remove an insulating sleeve 200 from the electrosurgical instrument 100. The removal of the insulating sleeve 200 may be performed without the use of the applicator 600. The steps involved in removing the insulating sleeve 200 from the electrosurgical instrument 100 are illustrated in figure 9.

The removal process begins at step 9A where the first and second parts 502, 504 of the housing 402 are closed around the shaft 102 of the electrosurgical instrument 100 by a user. The electrosurgical instrument 100 is therefore positioned within the housing 402. The insulating sleeve 200 is positioned on the shaft 102 and located distally of the second end of the housing 402. At step 9B the shaft 102 is held stationary and the housing 402 is progressed by the user towards the distal end of the electrosurgical instrument 100, i.e. towards the end effector 104.

At step 9C the support structure 410 of the housing 402 interfaces with the first end 202 of the insulating sleeve 200. Where the support structure 410 comprises a first 526 and second set 528 of protrusions as described above, a number of portions of the first end 202 of the insulating sleeve 200 interface with the distal ends of the protrusions of the first set of protrusions 526. Where the first set of protrusions 526 comprises two protrusions, two portions of the first end 202 of the insulating sleeve 200 interface with the distal ends of these protrusions. The tips of the protrusions apply a resistive force to the interfacing portions of the insulating sleeve 200. The portions of the insulating sleeve 200 that interface with the distal end of the protrusions are therefore caught on the distal end of the protrusions and move with the housing 402 as it is advanced further towards the end effector 104.

At step 9D a force is applied to the housing 402 such that it is advanced further along the electrosurgical instrument 100, and a number of further portions of the first end 202 of the insulating sleeve 200 interface with the distal ends of the protrusions of the second set of protrusions. Where the second set of protrusions 528 comprises two protrusions, two portions of the first end 202 of the insulating sleeve interface 200 with the distal ends of the protrusions. Thus, there are now twice the number of portions of the first end 202 of the insulating sleeve 200 to which a resistive force is applied. The user therefore requires less force to continue to progress the housing 402 towards the end effector 104.

At step 9E a force is continually applied until the electrosurgical instrument 100 is moved past the tips of the protrusions of the support structure 410. The insulating sleeve 200 continues to move with the housing 402, whilst the shaft 102 of the instrument 100 remains stationary. Thus, the insulating sleeve 200 is separated from the electrosurgical instrument 100 as the housing 402 reaches the end effector 104. The insulating sleeve 200 can then be discarded.

In view of the foregoing description it will be evident to a person skilled in the art that various modifications may be made within the scope of the appended claims.

## Claims

1. A device for applying an insulating sleeve (200) to an electrosurgical instrument (100), the device comprising a housing (402), the housing comprising:
a first end (404);
a second end (406);
a hollow channel (408) extending from the first end to the second end, the channel being configured to receive an electrosurgical instrument comprising an end effector (104) connected to a shaft (102) by a distal articulation (106);
a support structure (410) internal to the channel, wherein the proximal end of the support structure is located on a ledge (520) that separates a first portion (514) of the channel from a second portion (516) of the channel, the support structure being configured to:
support an insulating sleeve such that the insulating sleeve surrounds the electrosurgical instrument when the electrosurgical instrument is received by the channel; and
when the electrosurgical instrument is advanced through the channel from the first end to the second end, release the insulating sleeve onto the electrosurgical instrument.

2. A device as claimed in claim 1, wherein the first portion of the channel is narrower than the second portion of the channel.

3. A device as claimed in claim 1 or claim 2, wherein the first portion of the channel is circular in cross section, the first portion further comprising an opening (522) located on the ledge of the support structure.

4. A device as claimed in claim 3, wherein the support structure comprises a plurality of protrusions (524) surrounding the opening, the plurality of protrusions extending distally along an axis that is parallel to an axis (518) along which the channel extends, wherein the plurality of protrusions comprises a first set of protrusions (526) and a second set of protrusions (528), and wherein:
each protrusion of the first set of protrusions extends to a first length; and
each protrusion of the second set of protrusions extends to a second length that is shorter than the first length.

5. A device as claimed in claim 4, wherein the length of each protrusion of the second set of protrusions is between 45 to 50% of the length of each protrusion of the first set of protrusion.

6. A device as claimed in claim 4, wherein the protrusions of the support structure are uniformly positioned around the opening.

7. A device as claimed in any of claims 4 to 6, wherein each of the first and second sets of protrusions comprises two diametrically opposing protrusions, wherein the protrusions are angled such that the distance between two diametrically opposing protrusions is smaller than the shaft of the electrosurgical instrument.

8. A device as claimed in any preceding claim, wherein the housing is formed of a first portion and a second portion, the first portion and the second portion being pivotally coupled.

9. A device as claimed in claim 8, the device further comprising a hinge (506) coupled to both the first portion and the second portion, the hinge enabling pivotal rotation of the first portion and the second portion of the housing relative to each other.

10. A device as claimed in any preceding claim, wherein the device further comprises a removeable applicator (600), and wherein the housing is further configured to receive the applicator, the applicator comprising:
a first end (602) that can be coupled to the second end of the device;
a second end (604) that can be positioned at the first end of the device and configured to receive an end effector (104); and
a shaft (606) extending between the first end and the second end.

11. A device as claimed in claim 10, wherein the first end of the applicator comprises a laterally extending member (608) for releasably engaging the first end of the housing.

12. A device as claimed in claim 10 or claim 11, wherein the end effector is a pair of forceps and the second end of the applicator comprises an aperture (618) for housing the tips of the forceps when they are closed.

13. A device as claimed in any of claims 10 to 12, wherein the diameter of the shaft of the applicator is smaller than the diameter of the shaft of the electrosurgical instrument.

14. A device as claimed in any of claims 10 to 13, wherein the shaft of the applicator comprises a limiting ring (620), and wherein the diameter of the limiting ring is larger than the inner diameter of the insulating sleeve.

15. A method for applying an insulating sleeve (200) to an electrosurgical instrument (100), the electrosurgical instrument comprising an end effector (104) connected to a shaft (102) by a distal articulation (106) at a distal end of the instrument, the method comprising:
applying the insulating sleeve to a support structure (410) of a device, the device comprising a housing with a first end (404), a second end (406), and a hollow channel (408) extending from the first end to the second end, the support structure being internal to the channel and configured to interface with the insulating sleeve, the proximal end of the support structure being located on a ledge (520) that separates a first portion (514) of the channel from a second portion (516) of the channel;
progressing the shaft of the electrosurgical instrument through the hollow channel of the device; and
engaging the shaft of the electrosurgical instrument with the support structure, which pushes the insulating sleeve off of the support structure and onto the electrosurgical instrument.

## Patentansprüche

1. Vorrichtung zum Anbringen einer Isolierhülse (200) an einem elektrochirurgischen Instrument (100), wobei die Vorrichtung ein Gehäuse (402) umfasst, das Gehäuse umfassend:
ein erstes Ende (404);
ein zweites Ende (406);
einen hohlen Kanal (408), der sich von dem ersten Ende zu dem zweiten Ende erstreckt, wobei der Kanal konfiguriert ist, ein elektrochirurgisches Instrument aufzunehmen, das einen Endeffektor (104) umfasst, der mit einem Schaft (102) durch ein distales Gelenk (106) verbunden ist;
eine Stützstruktur (410) innerhalb des Kanals, wobei das proximale Ende der Stützstruktur auf einer Leiste (520) angeordnet ist, die einen ersten Abschnitt (514) des Kanals von einem zweiten Abschnitt (516) des Kanals trennt, wobei die Stützstruktur konfiguriert ist, um:
eine Isolierhülse so zu stützen, dass die Isolierhülse das elektrochirurgische Instrument umgibt, wenn das elektrochirurgische Instrument von dem Kanal aufgenommen wird; und
wenn das elektrochirurgische Instrument durch den Kanal von dem ersten Ende zu dem zweiten Ende vorgeschoben wird, die Isolierhülse auf das elektrochirurgische Instrument freizugeben.

2. Vorrichtung nach Anspruch 1, wobei der erste Abschnitt des Kanals schmaler als der zweite Abschnitt des Kanals ist.

3. Vorrichtung nach Anspruch 1 oder Anspruch 2, wobei der erste Abschnitt des Kanals einen kreisförmigen Querschnitt aufweist, wobei der erste Abschnitt ferner eine Öffnung (522) umfasst, die an der Leiste der Stützstruktur angeordnet ist.

4. Vorrichtung nach Anspruch 3, wobei die Stützstruktur eine Vielzahl von Vorsprüngen (524) umfasst, die die Öffnung umgeben, wobei die Vielzahl von Vorsprüngen sich distal entlang einer Achse erstreckt, die parallel zu einer Achse (518) ist, entlang der sich der Kanal erstreckt, wobei die Vielzahl von Vorsprüngen einen ersten Satz von Vorsprüngen (526) und einen zweiten Satz von Vorsprüngen (528) umfasst, und wobei:
jeder Vorsprung des ersten Satzes von Vorsprüngen sich auf eine erste Länge erstreckt; und
jeder Vorsprung des zweiten Satzes von Vorsprüngen sich auf eine zweite Länge erstreckt, die kürzer als die erste Länge ist.

5. Vorrichtung nach Anspruch 4, wobei die Länge jedes Vorsprungs des zweiten Satzes von Vorsprüngen zwischen 45 und 50 % der Länge jedes Vorsprungs des ersten Satzes von Vorsprüngen beträgt.

6. Vorrichtung nach Anspruch 4, wobei die Vorsprünge der Stützstruktur gleichmäßig um die Öffnung herum angeordnet sind.

7. Vorrichtung nach einem der Ansprüche 4 bis 6, wobei jeder der ersten und zweiten Sätze von Vorsprüngen zwei diametral gegenüberliegende Vorsprünge aufweist, wobei die Vorsprünge so abgewinkelt sind, dass der Abstand zwischen zwei diametral gegenüberliegenden Vorsprüngen kleiner als der Schaft des elektrochirurgischen Instruments ist.

8. Vorrichtung nach einem der vorhergehenden Ansprüche, wobei das Gehäuse aus einem ersten Abschnitt und einem zweiten Abschnitt gebildet ist, wobei der erste Abschnitt und der zweite Abschnitt schwenkbar gekoppelt sind.

9. Vorrichtung nach Anspruch 8, wobei die Vorrichtung ferner ein Scharnier (506) umfasst, das sowohl mit dem ersten Abschnitt als auch mit dem zweiten Abschnitt gekoppelt ist, wobei das Scharnier eine Schwenkdrehung des ersten Abschnitts und des zweiten Abschnitts des Gehäuses relativ zueinander ermöglicht.

10. Vorrichtung nach einem der vorhergehenden Ansprüche, wobei die Vorrichtung ferner einen abnehmbaren Applikator (600) umfasst, und wobei das Gehäuse ferner konfiguriert ist, den Applikator aufzunehmen, der Applikator umfassend:
ein erstes Ende (602), das mit dem zweiten Ende der Vorrichtung gekoppelt werden kann;
ein zweites Ende (604), das an dem ersten Ende der Vorrichtung positioniert werden kann und konfiguriert ist, einen Endeffektor (104) aufzunehmen; und
einen Schaft (606), der sich zwischen dem ersten Ende und dem zweiten Ende erstreckt.

11. Vorrichtung nach Anspruch 10, wobei das erste Ende des Applikators ein sich seitlich erstreckendes Element (608) zum lösbaren Eingriff mit dem ersten Ende des Gehäuses umfasst.

12. Vorrichtung nach Anspruch 10 oder Anspruch 11, wobei der Endeffektor ein Zangenpaar ist und das zweite Ende des Applikators eine Öffnung (618) zum Aufnehmen der Spitzen der Zangen umfasst, wenn diese geschlossen sind.

13. Vorrichtung nach einem der Ansprüche 10 bis 12, wobei der Durchmesser des Schafts des Applikators kleiner ist als der Durchmesser des Schafts des elektrochirurgischen Instruments.

14. Vorrichtung nach einem der Ansprüche 10 bis 13, wobei der Schaft des Applikators einen Begrenzungsring (620) umfasst und wobei der Durchmesser des Begrenzungsrings größer als der Innendurchmesser der Isolierhülse ist.

15. Verfahren zum Anbringen einer Isolierhülse (200) an einem elektrochirurgischen Instrument (100), wobei das elektrochirurgische Instrument einen Endeffektor (104) umfasst, der mit einem Schaft (102) durch ein distales Gelenk (106) an einem distalen Ende des Instruments verbunden ist, das Verfahren umfassend:
Anbringen der Isolierhülse an einer Stützstruktur (410) einer Vorrichtung, wobei die Vorrichtung ein Gehäuse mit einem ersten Ende (404), einem zweiten Ende (406) und einem hohlen Kanal (408) umfasst, der sich von dem ersten Ende zu dem zweiten Ende erstreckt, wobei die Stützstruktur im Inneren des Kanals angeordnet ist und konfiguriert ist, mit der Isolierhülse zusammenzuwirken, wobei das proximale Ende der Stützstruktur auf einer Leiste (520) angeordnet ist, die einen ersten Abschnitt (514) des Kanals von einem zweiten Abschnitt (516) des Kanals trennt;
Vorschieben des Schafts des elektrochirurgischen Instruments durch den hohlen Kanal der Vorrichtung; und
in Eingriff Bringen des Schafts des elektrochirurgischen Instruments mit der Stützstruktur, wodurch die Isolierhülse von der Stützstruktur auf das elektrochirurgische Instrument geschoben wird.

## Revendications

1. Dispositif pour appliquer un manchon isolant (200) à un instrument électrochirurgical (100), le dispositif comprenant un boîtier (402), le boîtier comprenant :
une première extrémité (404) ;
une deuxième extrémité (406) ;
un canal creux (408) s'étendant de la première extrémité à la deuxième extrémité, le canal étant configuré pour recevoir un instrument électrochirurgical comprenant un effecteur d'extrémité (104) raccordé à une tige (102) par une articulation distale (106) ;
une structure de support (410) interne au canal, dans laquelle l'extrémité proximale de la structure de support est située sur un rebord (520) qui sépare une première partie (514) du canal d'une deuxième partie (516) du canal, la structure de support étant configurée pour :
maintenir un manchon isolant de sorte que le manchon isolant entoure l'instrument électrochirurgical lorsque l'instrument électrochirurgical est reçu par le canal ; et
lorsque l'instrument électrochirurgical est avancé à travers le canal de la première extrémité à la deuxième extrémité, relâcher le manchon isolant sur l'instrument électrochirurgical.

2. Dispositif selon la revendication 1, dans lequel la première partie du canal est plus étroite que la deuxième partie du canal.

3. Dispositif selon la revendication 1 ou la revendication 2, dans lequel la première partie du canal est de section transversale circulaire, la première partie comprenant en outre une ouverture (522) située sur le rebord de la structure de support.

4. Dispositif selon la revendication 3, dans lequel la structure de support comprend une pluralité de saillies (524) entourant l'ouverture, la pluralité de saillies s'étendant distalement le long d'un axe qui est parallèle à un axe (518) le long duquel le canal s'étend, dans lequel la pluralité de saillies comprend un premier ensemble de saillies (526) et un deuxième ensemble de saillies (528), et dans lequel :
chaque saillie du premier ensemble de saillies s'étend sur une première longueur ; et
chaque saillie du deuxième ensemble de saillies s'étend sur une deuxième longueur qui est plus courte que la première longueur.

5. Dispositif selon la revendication 4, dans lequel la longueur de chaque saillie du deuxième ensemble de saillies est comprise entre 45 et 50 % de la longueur de chaque saillie du premier ensemble de saillies.

6. Dispositif selon la revendication 4, dans lequel les saillies de la structure de support sont positionnées uniformément autour de l'ouverture.

7. Dispositif selon l'une quelconque des revendications 4 à 6, dans lequel chacun des premier et deuxième ensembles de saillies comprend deux saillies diamétralement opposées, dans lequel les saillies sont inclinées de telle sorte que la distance entre deux saillies diamétralement opposées est inférieure au diamètre de la tige de l'instrument électrochirurgical.

8. Dispositif selon l'une quelconque des revendications précédentes, dans lequel le boîtier est formé d'une première partie et d'une deuxième partie, la première partie et la deuxième partie étant couplées de manière pivotante.

9. Dispositif selon la revendication 8, le dispositif comprenant en outre une charnière (506) couplée à la fois à la première partie et à la deuxième partie, la charnière permettant une rotation pivotante de la première partie et de la deuxième partie du boîtier l'une par rapport à l'autre.

10. Dispositif selon l'une quelconque des revendications précédentes, dans lequel le dispositif comprend en outre un applicateur amovible (600), et dans lequel le boîtier est en outre configuré pour recevoir l'applicateur, l'applicateur comprenant :
une première extrémité (602) qui peut être couplée à la deuxième extrémité du dispositif ;
une deuxième extrémité (604) qui peut être positionnée au niveau de la première extrémité du dispositif et configurée pour recevoir un effecteur d'extrémité (104) ; et
un arbre (606) s'étendant entre la première extrémité et la deuxième extrémité.

11. Dispositif selon la revendication 10, dans lequel la première extrémité de l'applicateur comprend un élément (608) s'étendant latéralement pour engager de manière amovible la première extrémité du boîtier.

12. Dispositif selon la revendication 10 ou la revendication 11, dans lequel l'effecteur d'extrémité est une paire de pinces et la deuxième extrémité de l'applicateur comprend une ouverture (618) pour loger les extrémités des pinces lorsqu'elles sont fermées.

13. Dispositif selon l'une quelconque des revendications 10 à 12, dans lequel le diamètre de la tige de l'applicateur est inférieur au diamètre de la tige de l'instrument électrochirurgical.

14. Dispositif selon l'une quelconque des revendications 10 à 13, dans lequel la tige de l'applicateur comprend une bague de limitation (620), et dans lequel le diamètre de la bague de limitation est plus grand que le diamètre intérieur du manchon isolant.

15. Procédé pour appliquer un manchon isolant (200) à un instrument électrochirurgical (100), l'instrument électrochirurgical comprenant un effecteur d'extrémité (104) raccordé à une tige (102) par une articulation distale (106) à une extrémité distale de l'instrument, le procédé comprenant :
l'application du manchon isolant sur une structure de support (410) d'un dispositif, le dispositif comprenant un boîtier avec une première extrémité (404), une deuxième extrémité (406), et un canal creux (408) s'étendant de la première extrémité à la deuxième extrémité, la structure de support étant interne au canal et configurée pour servir d'interface avec le manchon isolant, l'extrémité proximale de la structure de support étant située sur un rebord (520) qui sépare une première partie (514) du canal d'une deuxième partie (516) du canal ;
la progression de la tige de l'instrument électrochirurgical à travers le canal creux du dispositif ; et
la mise en prise de la tige de l'instrument électrochirurgical avec la structure de support, ce qui pousse le manchon isolant hors de la structure de support et sur l'instrument électrochirurgical.
